# FASCICULE DE BREVET EUROPEEN

(11) **EP 2 641 088 B1**
(45) Date de publication et mention de la délivrance du brevet: **28.10.2015**
(21) Numéro de dépôt: 11794821.6
(22) Date de dépôt: 16.11.2011
(51) Int. Cl.: G01N 33/558, G01N 33/543, G01N 33/569, G01N 33/576, G01N 33/68

(54) **DISPOSITIF ET PROCÉDÉ POUR IMMUNOESSAIS**
VORRICHTUNG UND VERFAHREN FÜR IMMUNOASSAYS
DEVICE AND METHOD FOR IMMUNOASSAYS

(30) Priorité: 17.11.2010 FR 1059431
(43) Date de publication de la demande: 25.09.2013
(73) Titulaire: Biomérieux, 69280 Marcy L'etoile (FR)
(72) Inventeur: BRIAND, Hélène, F-69210 L'Arbresle (FR); COLIN, Bruno, F-69280 Marcy L'etoile (FR); PARIS, Cécile, F-69690 Bessenay (FR)
(86) Numéro de dépôt international: PCT/FR2011/052663
(87) Numéro de publication internationale: WO 2012/066235

(56) Documents cités:
- EP-A1- 1 879 028
- EP-A2- 2 031 376
- WO-A1-02/01229
- WO-A2-2008/073222
- US-A1- 2007 243 630
- US-A1- 2008 032 420

## Description

La présente invention a pour objet un dispositif et un procédé pour réaliser un essai dénommé dosage à flux latéral pour déterminer la présence ou l'absence d'un analyte dans un échantillon. En particulier, l'invention se rapporte à de nouveaux contrôles améliorés de bon fonctionnement du dispositif.

Les dosages à flux latéral, encore appelés tests rapides, sont utilisés couramment dans les domaines d'analyses cliniques, alimentaires, pharmaceutiques et chimiques. Ainsi, les dispositifs de tests rapides sont utilisés pour déterminer la présence d'un grand nombre d'analytes, tels que des anticorps, des antigènes, des hormones, des protéines, des molécules chimiques dans des échantillons liquides. Ces dispositifs généralement comprennent un support et une matrice qui permet la migration de l'échantillon liquide. On distingue classiquement plusieurs zones au niveau de la matrice qui sont une zone d'application de l'échantillon liquide, une zone de marquage et une zone réactionnelle, cette dernière comprenant une zone de capture et une zone de contrôle. Ces différentes zones sont en communication de fluide. Ainsi, l'analyte à détecter, s'il est présent dans l'échantillon déposé au niveau de la zone d'application, se lie à un premier partenaire de liaison marqué au niveau de la zone de marquage, le complexe ainsi formé migre ensuite jusqu'à la zone réactionnelle où il est immobilisé au niveau de la zone de capture par réaction avec un second partenaire de liaison et l'utilisateur peut déterminer si l'analyte est bien présent par mise en évidence d'un signal détectable qui est déterminé par le type de marqueur associé au premier partenaire de liaison. Généralement, la présence de l'analyte dans l'échantillon est matérialisée sous la forme d'une ligne détectable, habituellement dénommée ligne test. La zone réactionnelle comprend également une zone de contrôle de migration de l'échantillon qui va indiquer à l'utilisateur qu'au moins une partie de l'échantillon est bien passée au travers de la matrice, en amont de la zone de contrôle et en particulier au niveau de la zone de capture. Ce peut être par exemple par la révélation d'une ligne de contrôle d'une couleur prédéterminée. On peut citer à titre d'exemple les demandes de brevet WO 2004/003559, WO 2006/092103, WO 2007/081330, US 2004/0161859. Les limites des moyens de contrôle actuellement utilisés dans les tests rapides sur bandelette, intégrée ou non dans une cassette, sont qu'ils ne peuvent que vérifier que la migration du fluide s'est bien effectuée par capillarité de la zone d'application jusqu'à la zone réactionnelle et ne peuvent pas contrôler le bon fonctionnement du dispositif et de l'essai.

Ainsi les demandes de brevet EP 2 031 376 et US 2008/0324230 décrivent un dispositif de dosage pour déterminer la présence ou l'absence d'un analyte dans un échantillon liquide. Avec ce dispositif, deux essais peuvent être effectués en parallèle avec possibilité d'intégrer une zone de contrôle de migration à ce dispositif sur l'une des deux bandes. La zone de contrôle de migration, lorsqu'elle est présente, est située en aval de la zone de détection. Chaque bande possède sa propre zone de marquage.

La demande de brevet US 2007/243630 décrit un dispositif de quantification d'un analyte dans un échantillon. Cette demande propose d'utiliser un analogue d'un analyte mais dans un rôle de barrière pour permettre la quantification de l'analyte, pas dans un rôle de contrôle.

La demande de brevet WO2008/073222 décrit un dispositif immunochromatographique impliquant une réaction type sandwich comprenant une ligne de contrôle de migration située en aval de la zone de détection. Ce dispositif ne comporte qu'une seule bande pour un seul dosage d'un analyte.

La demande de brevet EP 1 879 028 décrit un dispositif de dosage immunologique pour déterminer la présence ou la quantité d'un glucide bactérien dans un échantillon. Le dispositif tel que décrit comprend une zone de contrôle biologique comprenant un analogue de l'analyte. La zone de contrôle se trouve en aval de la zone de détection. Il ne comporte qu'une seule bande pour un seul dosage d'un analyte.

La demande de brevet WO02/01229 décrit un dispositif d'analyse chromatographique permettant un dosage immunologique unidirectionnel d'un analyte dans un échantillon de sang total. Le dispositif tel que décrit comprend une zone de contrôle qui se trouve en aval de la zone de détection. Ce dispositif peut être utilisé pour des dosages multiplexes permettant d'effectuer au moins deux dosages en même temps. Il comprend alors au moins deux zones d'application comprenant chacune sa propre zone de marquage.

La présente invention maintenant fournit un dispositif qui intègre un vrai contrôle positif. Le contrôle positif de l'invention permet de vérifier d'une part l'intégrité et le fonctionnement des éléments physiques du dispositif et d'autre part de vérifier la fonctionnalité des éléments biologiques du dispositif, en vue de contrôler le bon fonctionnement du dispositif et de l'essai.

Le dispositif de l'invention comprend :
- a) un support,
- b) une matrice 1, fixée sur le support, qui permet la migration de l'échantillon liquide, ladite matrice comprenant :
   - (i) une zone d'application de l'échantillon liquide 2,
   - (ii) une zone de marquage 3 comprenant au moins un premier partenaire de liaison marqué qui est susceptible de se lier audit au moins un analyte, s'il est présent dans l'échantillon liquide, et qui est susceptible de se lier à au moins un analogue de l'analyte, et
   - (iii) au moins une zone réactionnelle 4 comprenant :
- une zone de visualisation des résultats de l'essai 5 comprenant au moins un second partenaire de liaison immobilisé qui est susceptible de se lier audit au moins un analyte, et
- une zone de contrôle 6 en aval de la zone de visualisation des résultats 5 ou parallèle à la zone de visualisation des résultats de l'essai 5 qui permet de contrôler le fonctionnement du dispositif et qui comprend au moins un analogue du au moins un analyte qui est susceptible de se lier audit au moins premier partenaire de liaison marqué ;
- lesdites zones d'application de l'échantillon liquide 2, de marquage 3 et réactionnelle 4 étant en communication de fluide.

Le premier partenaire de liaison et le second partenaire de liaison sont choisis dans le groupe consistant en anticorps, mélange d'anticorps, fragment d'anticorps, mélange de fragments d'anticorps, analogue d'anticorps, mélange d'analogues d'anticorps, antigène, mélange d'antigènes, protéine, mélange de protéines, polypeptide, mélange de polypeptides, peptide, mélange de peptides.

L'analogue de l'analyte est soit immobilisé au niveau de la zone de contrôle directement ou indirectement, soit est susceptible, au niveau de la zone de contrôle, d'être entraîné par le flux de l'échantillon liquide jusqu'à une région déterminée de la zone de contrôle au niveau de laquelle il est immobilisé pour matérialiser la ligne de contrôle.

En conséquence, dans un mode de réalisation du dispositif de l'invention, la zone de contrôle 6 comprend de plus un réactif de capture immobilisé sur la matrice dudit au moins un analogue auquel ledit au moins un analogue est susceptible de se lier. En particulier, le réactif de capture dudit au moins un analogue est un réactif qui est identique au second partenaire de liaison de la zone de visualisation des résultats de l'essai.

Dans un autre mode de réalisation du dispositif de l'invention, dans la zone de contrôle 6 l'analogue de l'analyte est immobilisé sur la matrice directement ou indirectement.

L'analogue de l'analyte peut ainsi être immobilisé sur la matrice par un réactif choisi dans le groupe consistant en en anticorps, mélange d'anticorps, fragment d'anticorps, mélange de fragments d'anticorps, analogue d'anticorps, mélange d'analogues d'anticorps, antigène, mélange d'antigènes, protéine, mélange de protéines, polypeptide, mélange de polypeptides, peptide, mélange de peptides, récepteur, biotine/steptavidine, biotine/avidine.

L'analogue de l'analyte peut être aussi être immobilisé sur la matrice, par un réactif de capture qui est un réactif identique au second partenaire de liaison.

De préférence, l'analogue de l'analyte est un anticorps, un mélange d'anticorps, un fragment d'anticorps, un mélange de fragments d'anticorps, un analogue d'anticorps, un mélange d'analogues d'anticorps, un antigène, un mélange d'antigènes, une protéine, un mélange de protéines, un polypeptide, un mélange de polypeptides, un peptide, un mélange de peptides ou leurs associations.

Deux modes de réalisation préférés du dispositif selon l'invention sont décrits ci-dessous :
dans un mode de réalisation le premier partenaire de liaison est un anticorps, un fragment d'anticorps ou un analogue d'anticorps, le second partenaire de liaison est un anticorps, un fragment d'anticorps, ou un analogue d'anticorps et l'analogue de l'analyte est une protéine, un polypeptide ou un peptide, et
dans un autre mode de réalisation le premier partenaire de liaison est une protéine, un polypeptide ou un peptide, le second partenaire de liaison est une protéine, un polypeptide ou un peptide, et l'analogue de l'analyte est un anticorps, un fragment d'anticorps ou un analogue d'anticorps.

Le premier partenaire de liaison est marqué par un marqueur détectable, c'est à dire un composé, une substance ou une particule qui peut être détecté par des moyens visuels, fluorescents, instrumentaux et en particulier le marqueur détectable peut être une particule de latex coloré, une particule d'or, une particule magnétique.

Le bon fonctionnement du dispositif peut être visualisé par la formation d'une ligne de contrôle positif détectable qui est sensiblement perpendiculaire et de préférence perpendiculaire à la direction du flux de l'échantillon liquide.

Dans un autre mode de réalisation du dispositif selon l'invention, ce dernier comprend deux zones réactionnelles adjacentes et parallèles 4A et 4B qui ne sont pas en communication de fluide l'une par rapport à l'autre de telle sorte que la migration de l'échantillon liquide se fait simultanément et indépendamment dans lesdites zones. Il est souhaitable, dans ce mode de réalisation, que le dispositif comprenne de plus un contrôle de migration de l'échantillon liquide 7.

Dans les dispositifs précités la zone de contrôle (6) est de préférence en aval de la zone de visualisation des résultats (5).

Dans un autre mode de réalisation de l'invention, la zone réactionnelle 4 comprend deux zones réactionnelles adjacentes et parallèles (4A et 4B) qui ne sont pas en communication de fluide l'une par rapport à l'autre et la zone de contrôle (6) est parallèle à la zone de visualisation des résultats (5). Il est souhaitable dans ce mode de réalisation que le dispositif comprenne de plus un contrôle de migration de l'échantillon liquide (7).

Dans un autre mode de réalisation particulier du dispositif de l'invention, la matrice 1 est divisée en au moins deux parties 1A et 1B adjacentes et parallèles et qui ne sont pas en communication de fluide l'une par rapport à l'autre. La partie 1A comprend la zone d'application de l'échantillon liquide 2A, la zone marquage 3A, la zone de visualisation des résultats de l'essai 5, et de préférence la zone de contrôle de migration de l'échantillon 7. La partie 1B comprend la zone d'application de l'échantillon liquide 2B, la zone marquage 3B et la zone de contrôle 6 qui permet de contrôler le bon fonctionnement du dispositif.

Dans une variante du dispositif, la matrice 1 est divisée partiellement en deux parties adjacentes et parallèles l'une par rapport à l'autre. La matrice 1 comprend la zone d'application de l'échantillon liquide 2, la zone marquage 3. La zone réactionnelle 4 est divisée en deux parties adjacentes et parallèles et qui ne sont pas en communication de fluide l'une par rapport à l'autre. La zone 4A comprend la zone de visualisation des résultats de l'essai 5, et de préférence la zone de contrôle de migration de l'échantillon 7. La zone 4B comprend la zone de contrôle 6 qui permet de contrôler le bon fonctionnement du dispositif.

Dans un autre variante du dispositif de l'invention, la matrice 1 est divisée en au moins deux parties 1A et 1C adjacentes et parallèles qui ne sont pas en communication de fluide l'une par rapport à l'autre. La partie 1A comprend la zone d'application de l'échantillon liquide 2, la zone marquage 3A, la zone de visualisation des résultats de l'essai 5, et de préférence la zone de contrôle de migration de l'échantillon 7.La partie 1B comprend une zone d'application de l'analogue 9, la zone marquage 3C et la zone 6 qui permet de contrôler le bon fonctionnement du dispositif. Dans ce mode de réalisation, l'analogue de l'analyte peut être présent sous forme déshydratée auquel cas il est repris par tout moyen approprié, par exemple par un tampon ou par l'échantillon. L'analogue de l'analyte peut aussi être déposé au niveau de la zone d'application 9 sous forme liquide, en particulier après reprise par un milieu liquide approprié avant son dépôt au niveau de la zone d'application 9.

### DEFINITIONS

Le terme « matrice »fait référence à tout type de matériau qui est capable d'assurer le flux et le transfert d'un fluide. Le transfert du fluide peut être assuré par force de capillarité. La matrice peut être, par exemple en au moins un matériau bibuleux. Les matériaux bibuleux sont des matériaux qui facilement absorbent un liquide et au travers desquels le liquide est transporté par capillarité. A titre d'exemples non limitatifs on peut citer, comme matériau bibuleux, la nitrocellulose, le polyester, les fibres de verre, etc...

« Echantillon liquide » signifie tout échantillon prélevé chez un patient ou individu, et susceptible de contenir un analyte tel que défini ci-dessous. Cet échantillon peut être notamment un échantillon biologique liquide tel qu'un de sang, sérum, plasma, salive, urine, liquide céphalo-rachidien, liquide pleural, liquide péritonéal. Mais l'échantillon biologique comprend également les échantillons semi-solides ou solides dans la mesure où ils peuvent être transformés en échantillon liquide par toute méthode appropriée, par exemple un prélèvement alimentaire, un prélèvement de selles, un prélèvement de tissus, de cultures cellulaires, un prélèvement de muqueuses. On dispose de cet échantillon biologique par tout type de prélèvement connu de l'homme du métier. L'échantillon peut également être un échantillon d'origine environnementale, c'est à dire un échantillon liquide, solide ou semi-solide issu de l'environnement, tel que les effluents, les boues, les sols, les végétaux etc... Bien entendu, quand l'échantillon est solide ou semi-solide, il doit être prétraité pour être transformé en échantillon liquide.

« Analyte » signifie principalement, un antigène, un anticorps, une hormone, une protéine ou une molécule chimique.

Lorsque l'analyte est une protéine ou un antigène il peut être détecté par des partenaires de liaison, par exemple les récepteurs, anticorps, fragments d'anticorps, analogue d'anticorps et tout autre ligand capable de se lier à une protéine ou à un antigène.

Les anticorps partenaires de liaison sont par exemple soit des anticorps polyclonaux, soit des anticorps monoclonaux.

Les anticorps polyclonaux peuvent être obtenus par immunisation d'un animal avec l'immunogène approprié, suivie de la récupération des anticorps recherchés sous forme purifiée, par prélèvement du sérum dudit animal, et séparation desdits anticorps des autres constituants du sérum, notamment par chromatographie d'affinité sur une colonne sur laquelle est fixé un antigène spécifiquement reconnu par les anticorps.

Les anticorps monoclonaux peuvent être obtenus par la technique des hybridomes dont le principe général est rappelé ci-dessous.

Dans un premier temps, on immunise un animal, généralement une souris avec l'immunogène approprié, dont les lymphocytes B sont alors capables de produire des anticorps contre cet antigène. Ces lymphocytes producteurs d'anticorps sont ensuite fusionnés avec des cellules myélomateuses "immortelles" (murines dans l'exemple) pour donner lieu à des hybridomes. A partir du mélange hétérogène des cellules ainsi obtenu, on effectue alors une sélection des cellules capables de produire un anticorps particulier et de se multiplier indéfiniment. Chaque hybridome est multiplié sous la forme de clone, chacun conduisant à la production d'un anticorps monoclonal dont les propriétés de reconnaissance vis-à-vis de la protéine pourront être testées par exemple en ELISA, par immunotransfert (Western blot) en une ou deux dimensions, en immunofluorescence, ou à l'aide d'un biocapteur. Les anticorps monoclonaux ainsi sélectionnés, sont par la suite purifiés notamment selon la technique de chromatographie d'affinité décrite ci-dessus.

Les anticorps monoclonaux peuvent être également des anticorps recombinants obtenus par génie génétique, par des techniques bien connues de l'homme du métier.

Par « analogues d'anticorps » on entend des composés biologiques et/ou chimiques qui possèdent les mêmes capacités de liaison que les anticorps ou fragments d'anticorps ou des capacités de liaison similaires. En particulier, les analogues d'anticorps incluent de petites protéines qui comme les anticorps sont capables de se lier à une cible biologique permettant ainsi de la détecter, de la capturer ou tout simplement de la cibler au sein d'un organisme ou d'un échantillon biologique. Les champs d'applications de ces analogues d'anticorps sont pratiquement aussi vastes que ceux des anticorps. A titre d'exemple, on peut citer les Nanofitines™, petites protéines commercialisées par la société AFFILOGIC.

Les partenaires de liaison spécifiques de la protéine ou de l'antigène recherché dans le procédé de l'invention peuvent être utilisés comme réactif de capture, comme réactif de détection ou comme réactifs de capture et de détection.

La visualisation des réactions immunologiques, c'est-à-dire de la liaison protéine/partenaire de liaison ou antigène/partenaire de liaison, peut être effectuée par tout moyen de détection par l'intermédiaire d'un marquage, du partenaire de liaison.

Par marquage, on entend la fixation d'un réactif marqueur capable de générer un signal détectable, c'est à dire un composé, une substance ou une particule qui peut être détecté par des moyens visuels, fluorescents, instrumentaux.

Une liste non limitative de ces réactifs marqueurs consiste en :
- les particules métalliques ou d'alliages, telles que les particules d'or colloïdal,
- les particules de polymère, telles que les particules de latex colorés,
- les particules magnétiques,
- les molécules fluorescentes,
- les molécules chimioluminescentes.

Dans les modes de réalisation de l'invention, le signal généré au niveau de la zone de visualisation des résultats et le signal généré au niveau de la zone contrôle positif seront, de préférence, de la même nature et présenteront les mêmes couleurs.

A titre d'exemple de tests immunologiques tels que définis ci-dessus, on peut citer les méthodes « sandwich » et de « compétition ».

### FIGURES

**Figure 1** **:**

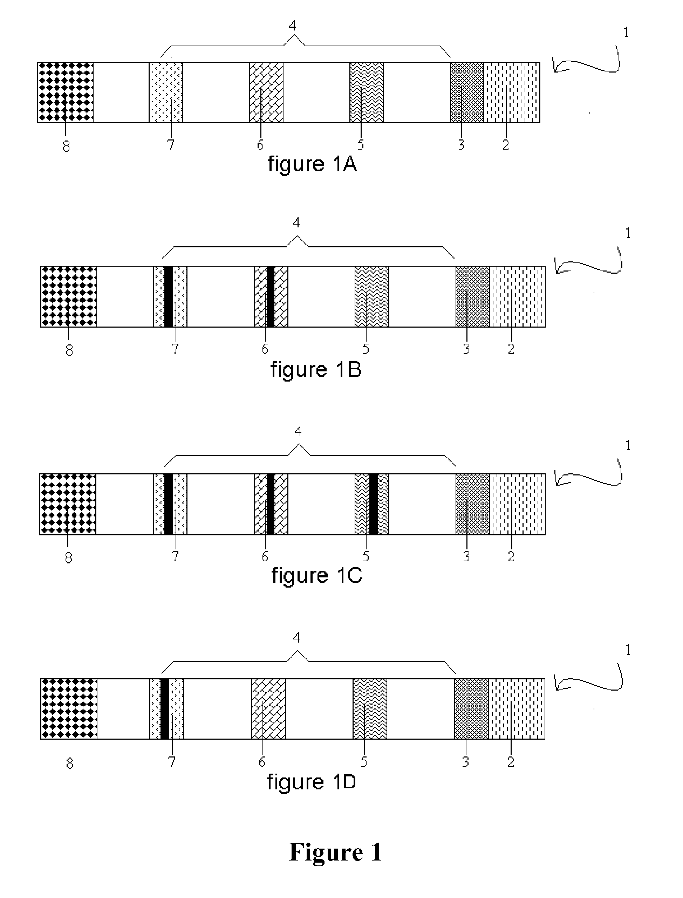

   La figure 1A est une vue de dessus d'un mode de réalisation du dispositif de l'invention, avant application de l'échantillon. Le dispositif de l'invention comprend un support (non représenté), une matrice 1 comprenant une zone d'application de l'échantillon 2, une zone de marquage 3, une zone réactionnelle 4 comprenant une zone de visualisation des résultats de l'essai 5 comprenant des moyens pour visualiser les résultats de l'essai et une zone de contrôle positif 6, en aval de la zone de visualisation des résultats de l'essai 5, comprenant des moyens pour effectuer le contrôle positif et des moyens pour visualiser le bon fonctionnement du dispositif et de l'essai. Optionnellement, la zone réactionnelle 4 peut de plus comprendre une zone de contrôle de migration 7 et des moyens pour visualiser la migration de l'échantillon. Facultativement, la matrice 1 peut également comprendre une zone d'absorption de l'échantillon 8.
   La figure 1B illustre les résultats obtenus avec le dispositif de la figure 1A après application d'un échantillon négatif pour un analyte à déterminer.
   La figure 1C illustre les résultats obtenus avec le dispositif de la figure 1A après application d'un échantillon positif pour un analyte à déterminer.
   La figure 1D illustre un cas particulier dans lequel l'utilisateur ne peut rendre de résultats.
   Bien qu'illustrées respectivement en 7 et 8 dans les figures 1B à 1D, les zones de contrôle de migration 7 et d'absorption 8 sont optionnelles dans ce mode de réalisation.
**Figure 2** **:**

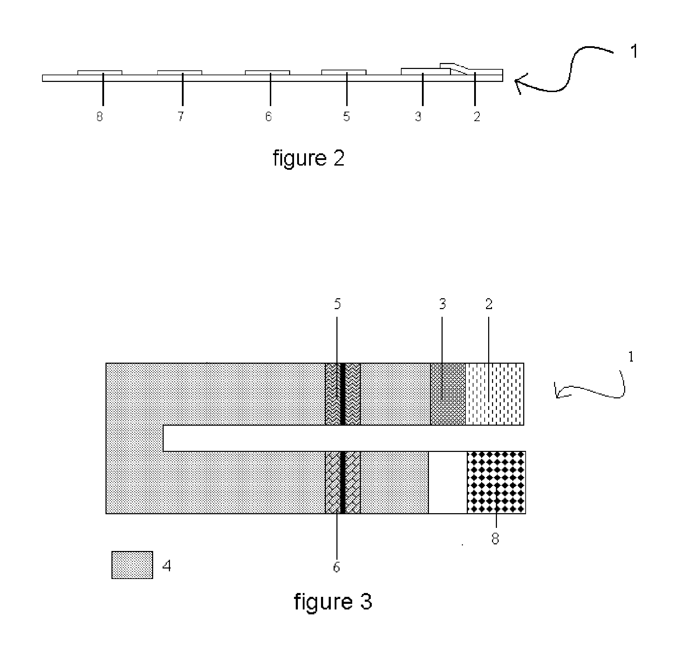

   La figure 2 est une vue de profil du dispositif de la figure 1A.
**Figure 3** **:**
   La figure 3 est une vue de dessus d'un mode de réalisation particulier du dispositif de l'invention, dans lequel la matrice 1 présente la forme d'un U couché. La matrice 1 comprend les moyens nécessaires pour la visualisation des résultats de l'essai au niveau de la zone 5 et des moyens pour effectuer le contrôle positif et pour visualiser le bon fonctionnement du dispositif et de l'essai. Dans la figure 3, la zone réactionnelle 4 est représentée en gris. La zone d'absorption 8 est facultative. La figure 3 illustre les résultats obtenus après application d'un échantillon positif pour un analyte à déterminer.
**Figure 4** **:**

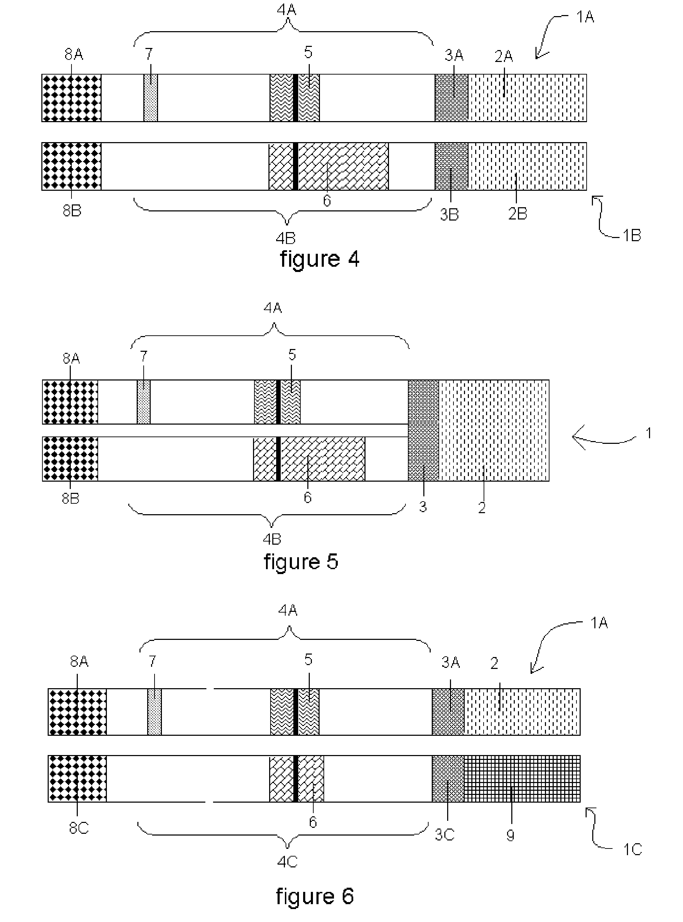

   La figure 4 illustre un mode de réalisation de l'invention, vu de dessus, dans lequel la matrice 1 est divisée en deux parties 1A et 1B adjacentes et parallèles et qui ne sont pas en communication de fluide l'une par rapport à l'autre. La matrice 1 comprend une zone d'application de l'échantillon 2 divisée en deux parties 2A et 2B, une zone de marquage divisée en deux parties 3A et 3B, une zone réactionnelle divisée en deux parties 4A et 4B. La zone réactionnelle 4A comprend la zone de visualisation des résultats de l'essai 5, une zone de contrôle de migration de l'échantillon 7 et les moyens pour visualiser les résultats de l'essai et pour visualiser la migration de l'échantillon. La zone réactionnelle 4B comprend la zone de contrôle positif 6, les moyens pour effectuer le contrôle positif et les moyens pour visualiser le bon fonctionnement du dispositif et de l'essai au niveau de la zone de contrôle 6. Les zones de visualisation des résultats de l'essai 5 et de contrôle 6 sont adjacentes et parallèles l'une par rapport à l'autre. Optionnellement, la matrice 1 comprend de plus une zone d'absorption 8 divisée en deux parties représentées respectivement en 8A et 8B. Les zones d'application de l'échantillon liquide 2, de marquage 3 et réactionnelle 4 sont en communication de fluide. La figure 4 illustre les résultats obtenus après application d'un échantillon positif pour un analyte à déterminer.
**Figure 5** **:**
   La figure 5 illustre un autre mode de réalisation du dispositif selon l'invention qui comporte deux zones réactionnelles 4A et 4B adjacentes et parallèles et qui ne sont pas en communication de fluide l'une par rapport à l'autre l'une par rapport à l'autre de telle sorte que la migration de l'échantillon liquide se fait simultanément et indépendamment dans lesdites zones. La matrice 1 comprend une zone d'application de l'échantillon 2, une zone de marquage 3, une zone réactionnelle 4 divisée en 2 parties 4A et 4B. La zone réactionnelle 4A comprend la zone de visualisation des résultats de l'essai 5, une zone de contrôle de migration de l'échantillon 7 et les moyens pour visualiser les résultats de l'essai et pour visualiser la migration de l'échantillon. La zone réactionnelle 4B comprend la zone de contrôle positif 6 et les moyens pour effectuer le contrôle positif et pour visualiser le bon fonctionnement du dispositif et de l'essai au niveau de la zone de contrôle 6. Les zones de visualisation des résultats de l'essai 5 et de contrôle 6 sont adjacentes et parallèles l'une par rapport à l'autre. Optionnellement la matrice 1 comprend de plus une zone d'absorption 8 divisée en deux parties 8A et 8B. Les zones d'application de l'échantillon liquide 2, de marquage 3 et réactionnelle 4 sont en communication de fluide. La figure 5 illustre les résultats obtenus après application d'un échantillon positif pour un analyte à déterminer.
**Figure 6** **:**
   La figure 6 illustre un mode de réalisation de l'invention dans lequel la matrice 1 est divisée en deux parties 1A et 1C adjacentes et parallèles et qui ne sont pas en communication de fluide l'une par rapport à l'autre l'une par rapport à l'autre. La matrice 1 comprend une zone d'application de l'échantillon 2, une zone de marquage divisée en deux parties 3A et 3C, une zone réactionnelle 4 divisée en deux paries 4A et 4B. La zone réactionnelle 4A comprend la zone de visualisation des résultats de l'essai 5, une zone de contrôle de migration de l'échantillon 7 et les moyens pour visualiser les résultats de l'essai et pour visualiser la migration de l'échantillon.
   La zone réactionnelle 4B comprend la zone 6 de contrôle et les moyens nécessaires pour effectuer le contrôle positif et pour visualiser le bon fonctionnement du dispositif et de l'essai. Les zones de visualisation des résultats de l'essai 5 et de contrôle 6 sont adjacentes et parallèles l'une par rapport à l'autre. La matrice 1, dans sa partie 1C, comprend une zone de dépôt de l'analogue 9.
   Facultativement la matrice 1 comprend une zone d'absorption 8 divisée en deux parties 8A et 8B. Les zones d'application de l'échantillon liquide 2, de marquage 3 et réactionnelle 4 sont en communication de fluide. La figure 6 illustre les résultats obtenus après application d'un échantillon positif pour un analyte à déterminer.

### MODES DE REALISATION

Dans un mode de réalisation, en référence à la figure 1, la matrice 1 est représentée sous la forme d'une bande rectangulaire dont l'axe longitudinal est en position horizontale. Les zones 2, 3 et 4 sont en communication de fluide. La zone 3 comprend le premier partenaire de liaison marqué, par exemple un anticorps portant un marqueur visible, tel qu'une particule de latex coloré, une particule d'or etc... Ce réactif peut migrer librement au travers de la matrice en présence de l'échantillon liquide déposé au niveau de la zone 2 et réagir avec l'analyte (antigène) à déterminer s'il est présent. Dans la zone 5 de la matrice 1, le second partenaire de liaison, par exemple un anticorps ayant une spécificité pour un épitope de l'antigène qui est différent de celui reconnu par le premier anticorps marqué, est immobilisé. Dans la zone 6 de la matrice 1, un analogue de l'antigène est soit immobilisé directement ou indirectement ou peut migrer librement en présence du flux de fluide au niveau de la zone 6 jusqu'à ce qu'il soit immobilisé par un réactif de capture qui est identique au second anticorps.

Les figures 1B et 1C illustrent le fonctionnement de l'essai en présence d'un contrôle négatif et d'échantillons positifs.

Comme illustré à la figure 1B, l'échantillon étant un contrôle négatif, il n'y a pas d'émission d'un signal détectable au niveau de la zone de visualisation des résultats 5. Par contre, il y a émission d'un signal détectable au niveau de la zone de contrôle positif 6, matérialisé, par exemple, par une ligne perpendiculaire au sens du flux de l'échantillon liquide, ce qui signifie d'une part que l'échantillon contrôle négatif a bien migré jusqu'à la zone 6 et que d'autre part le dispositif est fonctionnel.

Comme illustré à la figure 1C, l'échantillon étant positif, Il y a émission d'un signal détectable au niveau de la zone de visualisation des résultats 5 qui est matérialisé par une ligne perpendiculaire au sens du flux de l'échantillon liquide. Il y a également émission d'un signal détectable au niveau de la zone de contrôle positif 6, matérialisé, par exemple, par une ligne perpendiculaire au sens du flux de l'échantillon liquide, ce qui signifie d'une part que l'échantillon a bien migré et que d'autre part le dispositif est fonctionnel. L'intensité du signal au niveau des zones 5 et 6 sera fonction de la charge de l'échantillon, comme expliqué plus en détail dans les exemples qui suivent.

Comme illustré à la figure 1D, il n'y a pas d'émission de signal détectable ni au niveau de la zone 5 de visualisation des résultats de l'essai, ni au niveau de la zone de contrôle 6. Les résultats sont ininterprétables et l'essai doit être renouvelé. Dans le cas où un contrôle de migration est prévu au niveau de la zone 7, la visualisation d'un signal de migration permet de constater que ces résultats ne sont pas dus à un dysfonctionnement physique du dispositif.

Dans un autre mode de réalisation illustré à la figure 4, la matrice 1 est représentée sous la forme de deux bandes 1A et 1B, dont l'axe longitudinal est en position horizontale. Les deux bandes 1A et 1B sont adjacentes et parallèles et ne sont pas en communication de fluide l'une par rapport à l'autre. Les zones 2A, 3A et 4A sont en communication de fluide. Les zones 2B, 3B et 4B sont en communication de fluide. Les zones 3A et 3B comprennent respectivement le premier partenaire de liaison marqué, par exemple un anticorps portant un marqueur visible, tel qu'une particule de latex coloré, une particule d'or etc.... Ce réactif peut migrer librement au travers de la matrice 1A en présence de l'échantillon liquide déposé au niveau de la zone d'application 2A et réagir avec l'analyte (antigène) à déterminer s'il est présent. Dans la zone 5 de la matrice 1A, le second partenaire de liaison, par exemple un anticorps ayant une spécificité pour un épitope de l'antigène qui est différent de celui reconnu par le premier anticorps marqué, est immobilisé. Si l'antigène est présent, il y a émission d'un signal détectable au niveau de la zone de visualisation des résultats 5 qui se matérialise, par exemple, par une ligne perpendiculaire au sens du flux de l'échantillon liquide, comme illustré à la figure 4. De plus, que l'échantillon soit négatif ou positif, un signal de migration de l'échantillon doit apparaître au niveau de la zone 7 de contrôle de migration. La matrice 1B comprend de plus un analogue de l'antigène qui est soit immobilisé directement ou indirectement ou peut migrer librement en présence du flux de l'échantillon au niveau de la zone 6 jusqu'à ce qu'il soit immobilisé par un réactif de capture qui est identique au second anticorps.

Dans un autre mode de réalisation illustré à la figure 5, l'échantillon liquide est déposé au niveau de la zone d'application de l'échantillon 2 par tout moyen approprié. Après application, l'échantillon liquide commence à migrer au travers la matrice, entre en contact avec le premier partenaire de liaison marqué (anticorps marqué) au niveau de la zone de marquage 3 de façon à ce qu'un complexe analyte/premier partenaire de liaison marqué se forme si l'analyte est présent dans l'échantillon. Le complexe marqué migre avec le flux de l'échantillon respectivement jusqu'aux zones réactionnelles 4A et 4B. Le complexe marqué est d'une part immobilisé au niveau de la zone de visualisation de l'essai 5 par liaison avec le second partenaire de liaison, spécifique de l'analyte (antigène), qui est immobilisé dans cette zone, de sorte qu'un signal est généré au niveau de la zone de visualisation des résultats, de préférence sous la forme d'une ligne perpendiculaire au sens de déplacement de l'échantillon. Le fluide restant, comprenant le premier partenaire de liaison marqué et le complexe marqué migre d'autre part jusqu'à la zone 6 de contrôle comprenant un analogue de l'antigène qui est soit immobilisé directement ou indirectement au niveau de la matrice, soit qui est libre de migrer avec le flux de l'échantillon jusqu'à ce qu'il soit immobilisé au niveau de la zone 6 par un réactif de capture qui est identique au second anticorps, de sorte qu'un signal est généré au niveau de la zone de contrôle 6, de préférence sous forme d'une ligne perpendiculaire au sens de déplacement du fluide et parallèle à la ligne test.

Dans un autre mode de réalisation et par référence à la figure 6, la matrice 1 est composée de deux bandes 1A et 1B adjacentes et parallèles et qui ne sont pas en communication de fluide l'une par rapport à l'autre. Les zones 2A, 3A et 4A sont en communication de fluide. Les zones 2C, 3C et 4C sont en communication de fluide. Les zones 3A et 3B comprennent respectivement le premier partenaire de liaison marqué, par exemple un anticorps portant un marqueur visible, tel qu'une particule de latex coloré, une particule d'or etc.... Ce réactif peut migrer librement au travers de la matrice 1A en présence de l'échantillon liquide déposé au niveau de la zone d'application 2A et réagir avec l'analyte (antigène) à déterminer s'il est présent. Dans la zone 5 de la matrice 1A, le second partenaire de liaison, par exemple un anticorps ayant une spécificité pour un épitope de l'antigène qui est différent de celui reconnu par le premier anticorps marqué, est immobilisé. Si l'antigène est présent, il y a émission d'un signal détectable au niveau de la zone de visualisation des résultats 5 qui se matérialise, par exemple, par une ligne perpendiculaire au sens du flux de l'échantillon liquide. De plus, que l'échantillon soit négatif ou positif, un signal de migration de l'échantillon doit apparaître au niveau de la zone 7 de contrôle de migration. La matrice 1C comprend l'analogue de l'antigène qui est soit déposé sous forme liquide au niveau d'une zone de dépôt 9, soit est présent sous forme sèche au niveau de la zone 9 et est reconstitué par tout moyen approprié. La matrice 1C comprend de plus un analogue de l'antigène qui est soit immobilisé directement ou indirectement ou peut migrer librement en présence du flux de liquide au niveau de la zone 6 jusqu'à ce qu'il soit immobilisé par un réactif de capture qui est identique au second anticorps.

### EXEMPLES

### Exemple 1 - Dosage de l'antigène HBs

Le dosage de l'antigène HBs par l'essai illustré à la figure 1 consiste en une réaction immunologique de type sandwich en une étape basée sur une technique immunochromatographique.

Des particules latex rouges commercialisées par la société Magsphère (nom commercial) sont coatées par un mélange de deux anticorps monoclonaux anti-HBs (bioMérieux, 2G2G10A12 et 6H6B6), à une concentration respective de 500 µg/ml. Les particules sont ensuite réparties grâce à un appareil BIODOT (nom commercial) sur une membrane de polyester (Ahlstrom - nom commercial). La membrane est séchée une nuit à 37°C.

L'anticorps de capture est un polyclonal de chèvre anti-HBs, produit par bioMerieux, qui coaté sur une membrane de nitrocellulose CN 140 (Sartorius - nom commercial) à une concentration de 1mg/ml. La répartition est réalisée avec l'appareil BIODOT.

Le contrôle positif du test HBs Ag ou analogue de l'antigène natif HBs est un antigène HBs recombinant (lot 101011FFU04), développé par biomérieux, qui est immobilisé directement, au niveau de la zone de contrôle, à une concentration de 1 mg/ml par répartition avec une l'appareil BIODOT sur la membrane de nitrocellulose, à une distance de 5 mm par rapport à l'anticorps polyclonal anti-HBs de capture. Après répartition de l'anticorps de capture et du contrôle positif du test, la membrane est séchée une nuit à 37°C.

Les deux membranes de polyester et de nitrocellulose, sont ensuite assemblées sur un support de test rapide (backing, de la société G&L (nom commmercial)). Le montage dans les cassettes est réalisé après découpage sous forme de bandelette.

Les échantillons testés sont des échantillons positifs pour l'antigène HBs Ag bien caractérisés.

Des dilutions dans du sérum négatif (Scantibodies - nom commercial) sont réalisées pour obtenir des niveaux de positivité fort, moyen et faible. L'échantillon négatif testé correspond à un pool de sérum négatif en provenance de l'Etablissement Français du Sang (EFS) de la région Rhône-Alpes.

Le temps de lecture après dépôt de l'échantillon dans le puits de dépôt de l'échantillon de la cassette est de 15 minutes.

La lecture est réalisée visuellement à l'aide d'une carte de lecture qui permet d'attribuer des intensités de signal selon l'intensité de la couleur rouge observée.

Cette carte est graduée de L1 à L10. Un échantillon est considéré positif si une couleur rouge apparaît avec une intensité correspondant au minimum à L3 de l'échelle de lecture.

Les résultats sont présentés dans le tableau 1 ci-dessous :

**Tableau 1**

| | HBs Ag lot 101011FFU04 (bioMérieux) | |
|---|---|---|
| Echantillons | Ligne Test | Ligne Contrôle Positif |
| | 15 minutes | 15 minutes |
| Map60 (positif fort) | L7 | L7 |
| Map59 (positif fort) | L7 | L7 |
| Map60 (positif moyen) | L5 | L8 |
| Map59 (positif moyen) | L5 | L8 |
| Map64 (positif faible) | L4 | L8 |
| Map60 (positif faible) | L4 | L8 |
| Pool de sérum négatif | L1 | L8 |

Les résultats montrent que dans le cas d'un échantillon négatif, seul le contrôle positif est détecté avec une intensité de couleur forte (L8). Ce résultat permet de confirmer que l'absence de signal au niveau de la ligne test 5, qui correspond au polyclonal de capture, est lié à la négativité de l'échantillon et non à un défaut fonctionnel de la cassette utilisée pour le dosage. En effet, en cas d'un sérum négatif, les anticorps monoclonaux couplés aux particules rouges sont disponibles et un complexe est formé avec l'antigène HBs recombinant lors de la migration des particules au niveau de la ligne contrôle 6.

A l'inverse, en cas d'un échantillon positif, les particules fixant l'antigène présent dans l'échantillon forme un complexe avec le polyclonal de capture au niveau de la ligne test 5, selon le niveau de positivité de l'échantillon, des anticorps au niveau de la particule peuvent rester disponibles et viennent former un deuxième complexe au niveau de la ligne contrôle 6.

### Exemple 2 - Dosage de l'antigène de la grippe A

Le dosage de l'antigène de la grippe A est basé sur le même principe que celui décrit dans l'exemple 1 pour le dosage de l'antigène HBs Ag. Un anticorps monoclonal de détection (bioMérieux, 15C9H2) anti-grippe A est immobilisé sur des particules rouges (Magsphère). Le même anticorps est utilisé en capture sur la membrane de nitrocellulose. Le contrôle positif (analogue de l'antigène) est une protéine recombinante (Nucleoprotein Influenza A lot 101011FFU05, bioMérieux) immobilisée directement sur la membrane de nitrocellulose au niveau de la zone de contrôle, à une concentration de 1mg/ml. L'analogue est réparti par l'appareil BIODOT sur la membrane de nitrocellulose à une distance de 5 mm par rapport au monoclonal de capture anti-grippe A. Après assemblage et montage dans les cassettes, les dosages sont réalisés avec un temps de lecture à 10 minutes après dépôt de l'écahntillon.

Une gamme de concentration de la protéine recombinante (Nucleoprotein INF A, bioMérieux) a été testée. L'échantillon négatif testé est un tampon PBS.

Les résultats sont présentés dans le tableau 2 ci-dessous :

**Tableau 2**

| | Influenza A lot 101011FFU05 (bioMérieux) | |
|---|---|---|
| Echantillons | Ligne Test | Ligne Contrôle Positif |
| | 10 minutes | 10 minutes |
| NP A INF (40µg/ml) | L10 | L6 |
| NP A INF (1µg/ml) | L10 | L8 |
| NP A INF (50ng/ml) | L7 | L10 |
| NP A INF (10ng/ml) | L4 | L10 |
| Tampon bioMérieux lot 100923FFU03 | L1 | L10 |

Les résultats montrent que dans le cas d'un échantillon négatif, seul le contrôle positif est détecté avec une intensité de couleur forte (L10). Ce résultat permet de confirmer que l'absence de signal au niveau de la ligne test 5 (correspondant au monoclonal de capture), est liée à la négativité de l'échantillon et non à un défaut fonctionnel de la cassette utilisée pour le dosage. En effet, en cas d'un sérum négatif, les anticorps monoclonaux couplés aux particules rouges sont disponibles et un complexe est formé avec la protéine recombinante Influenza A lors de la migration des particules au niveau de la ligne contrôle 6. A l'inverse, en cas d'un échantillon positif, les particules fixant l'antigène présent dans l'échantillon forme un complexe avec le monoclonal de capture au niveau de la ligne test 5. Selon le niveau de positivité de l'échantillon testé (de 40 µg/ml à 10 ng/ml), des anticorps au niveau de la particule peuvent rester disponibles et viennent former un deuxième complexe au niveau de la ligne contrôle 6.

### Exemple 3 - Dosage d'anticorps anti-HIV-1 groupe M

La détection est basée sur les mêmes principes que ceux décrits dans les exemples 1 et 2, c'est à dire un dosage immunochromatographique de type sandwich en une étape. La seule différence réside dans le fait que dans cet essai on dose la présence d'un anticorps, c'est à dire un anticorps anti-HIV-1 groupe M

Des particules latex bleues commercialisées par la société VARIAN (nom commercial) sont coatées avec des peptides spécifiques du virus HIV-1 groupe M. Ces particules sont ensuite réparties sur une membrane de polyester (Ahlstrom). Les peptides de captures sont coatés sur la membrane de nitrocellulose (Millipore, 135UF).

Le contrôle positif du test HIV est un anticorps monoclonal anti-HIV-1 groupe M (bioMérieux, P12G11B10) coaté sur la membrane de nitrocellulose à une concentration de 1mg/ml. L'anticorps contrôle positif est réparti par l'appareil à une distance de 5 mm par rapport au peptides de capture.

Après répartition des peptides de capture et du contrôle positif du test, la membrane est séchée une nuit à 37°C.

Après assemblage et montage dans les cassettes, les dosages sont réalisés. La lecture du signal est effectuée à 30 minutes après le dépôt de l'échantillon.

Les échantillons testés sont des échantillons HIV positifs bien caractérisés. L'échantillon négatif est un pool de sérum négatif en provenance des EFS de la région Rhône Alpe.

Les résultats sont présentés dans le tableau 3 ci-dessous :

**Tableau 3**

| HIV 1/2 | HIV ½ lot 101011FFU03 | |
|---|---|---|
| | Ligne Test | Ligne Contrôle Positif |
| | Temps de lecture 30 minutes | Temps de lecture 30 minutes |
| MARHIV0023 (SCI13)HIV1 M | L10 | L1 |
| MARHIV0024 (SCI14)HIV1 M | L10 | L1 |
| MARHIV0025 (SCI15)HIV2 | L9 | L6 |
| MARHIV0032 (SCI22)HIV group O | L8 | L6 |
| Pool de sérum négatif | L1 | L6 |

Les résultats montrent que dans le cas d'un échantillon négatif, seul le contrôle positif est détecté. Ce résultat permet de confirmer que l'absence de signal au niveau de la ligne test 5 (correspondant aux peptides de capture), est liée à la négativité de l'échantillon et non à un défaut fonctionnel de la cassette utilisée pour le dosage. En effet, en cas d'un sérum négatif, les peptides HIV couplés aux particules bleues sont disponibles et un complexe est formé avec l'anticorps monoclonal anti-HIV de contrôle lors de la migration des particules au niveau de la ligne contrôle 6.

A l'inverse, en cas d'un échantillon positif, les particules fixant les anticorps anti-HIV présents dans l'échantillon forme un complexe avec les peptides de capture au niveau de la ligne test 5, selon le niveau de positivité de l'échantillon, les peptides au niveau de la particule sont saturées ou restent partiellement disponibles et viennent former un deuxième complexe au niveau de la ligne contrôle 6.

## Revendications

1. Dispositif pour réaliser un essai pour déterminer la présence ou l'absence d'au moins un analyte dans un échantillon liquide comprenant :
- a) un support,
- b) une matrice (1), fixée sur le support, qui permet la migration de l'échantillon liquide, ladite matrice comprenant :
- (i) une zone d'application de l'échantillon liquide (2),
- (ii) une zone de marquage (3) comprenant au moins un premier partenaire de liaison marqué qui est susceptible de se lier audit au moins un analyte, s'il est présent dans l'échantillon liquide, et qui est susceptible de se lier à au moins un analogue de l'analyte, et
- (iii) au moins une zone réactionnelle (4) comprenant deux zones réactionnelles (4A et 4B ou 4C) adjacentes et parallèles qui ne sont pas en communication de fluide l'une par rapport à l'autre, dans lesquelles :
- la zone réactionnelle (4A) comprend une zone de visualisation des résultats de l'essai (5) comprenant au moins un second partenaire de liaison immobilisé qui est susceptible de se lier audit au moins un analyte, et
- la zone réactionnelle (4B ou 4C) comprend une zone de contrôle (6) qui permet de contrôler le bon fonctionnement du dispositif et qui comprend au moins un analogue dudit au moins un analyte qui est susceptible de se lier audit au moins premier partenaire de liaison marqué ;
- lesdites zones d'application de l'échantillon liquide (2), de marquage (3) et réactionnelle (4) étant en communication de fluide.

2. Dispositif selon la revendication 1, dans lequel le premier partenaire de liaison et le second partenaire de liaison sont choisis dans le groupe consistant en anticorps, mélange d'anticorps, fragment d'anticorps, mélange de fragments d'anticorps, analogue d'anticorps, mélange d'analogues d'anticorps, antigène, mélange d'antigènes, protéine, mélange de protéines, polypeptide, mélange de polypeptides, peptide, mélange de peptides.

3. Dispositif selon la revendication 1, dans lequel la zone de contrôle (6) comprend de plus un réactif de capture de l'analogue, immobilisé sur la matrice, et auquel ledit un analogue est susceptible de se lier.

4. Dispositif selon la revendication 1, dans lequel dans la zone (6) l'analogue est immobilisé sur la matrice directement ou indirectement.

5. Dispositif selon la revendication 3, dans lequel le réactif de capture de l'analogue est un réactif qui est identique au second partenaire de liaison de la zone de visualisation des résultats de l'essai.

6. Dispositif selon la revendication 4, dans lequel l'analogue de l'analyte est immobilisé sur la matrice par un réactif choisi dans le groupe consistant en en anticorps, mélange(s) d'anticorps, fragment d'anticorps, mélange de fragments d'anticorps, analogue d'anticorps, mélange d'analogues d'anticorps, antigène, mélange d'antigènes, protéine, mélange de protéines, polypeptide et mélange de polypeptides, peptide, mélange de peptides, récepteur, biotine/steptavidine, biotine/avidine.

7. Dispositif selon la revendication 1, dans lequel l'analogue de l'analyte est choisi dans le groupe consistant en anticorps, mélange d'anticorps, fragment d'anticorps, mélange de fragments d'anticorps, analogue d'anticorps, mélange d'analogues d'anticorps, antigène, mélange d'antigènes, protéine, mélange de protéines, polypeptide, mélange de polypeptides, peptide, mélange de peptides.

8. Dispositif selon la revendication 1, dans lequel
(i) le premier partenaire de liaison est un anticorps, un fragment d'anticorps ou un analogue d'anticorps, le second partenaire de liaison est un anticorps, un fragment d'anticorps ou un analogue d'anticorps et l'analogue de l'analyte est une protéine, un polypeptide ou un peptide, ou
(ii) le premier partenaire de liaison est une protéine, un polypeptide ou un peptide, le second partenaire de liaison est une protéine, un polypeptide ou un peptide, et l'analogue de l'analyte est un anticorps, un fragment d'anticorps ou un analogue d'anticorps.

9. Dispositif selon la revendication 8, dans lequel l'analogue de l'analyte est immobilisé sur la matrice, par un réactif de capture qui est un réactif identique au second partenaire de liaison.

10. Dispositif selon la revendication 1, dans lequel le premier partenaire de liaison est marqué par un marqueur détectable.

11. Dispositif selon la revendication 10, dans lequel le marqueur détectable est un composé, une substance ou une particule qui peut être détecté par des moyens visuels, fluorescents, instrumentaux et en particulier le marqueur détectable pout être une particule de latex coloré, une particule d'or, une particule magnétique.

12. Dispositif selon l'une quelconque des revendications précédentes dans lequel le bon fonctionnement du dispositif et de l'essai est matérialisé sous la forme d'une ligne détectable qui est sensiblement perpendiculaire à la direction du flux de l'échantillon liquide.

13. Dispositif selon les revendications précédentes, comprenant de plus un contrôle de migration de l'échantillon liquide (7).

14. Procédé pour réaliser un essai pour déterminer la présence ou l'absence d'un analyte dans un échantillon liquide comprenant les étapes de :
mettre en contact l'échantillon liquide avec un dispositif tel que défini dans l'une quelconque des revendications précédentes et déterminer si l'échantillon ne comprend pas ou comprend l'analyte par référence à l'absence ou à la présence d'un signal détectable au niveau de la zone de visualisation des résultats de l'essai et quand il y a absence de signal au niveau de la zone de visualisation des résultats de l'essai confirmer le bon fonctionnement de l'essai par référence à la présence d'un signal au niveau de la zone de contrôle.

## Patentansprüche

1. Vorrichtung, um einen Assay zur Bestimmung der Gegenwart oder der Abwesenheit mindestens eines Analyten in einer flüssigen Probe auszuführen, umfassend:
a) einen Träger,
b) eine Matrix (1), die auf dem Träger fixiert ist, die die Migration der flüssigen Probe ermöglicht, wobei die Matrix Folgendes umfasst:
(i) eine Zone zum Auftragen der flüssigen Probe (2),
(ii) eine Markierungszone (3), die mindestens einen ersten markierten Verbindungspartner umfasst, der imstande ist, sich mit dem mindestens einen Analyten zu verbinden, wenn er in der flüssigen Probe vorhanden ist, und der imstande ist, sich mit mindestens einem Analogon des Analyten zu verbinden, und
(iii) mindestens eine Reaktionszone (4), die zwei benachbarte und parallele Reaktionszonen (4A und 4B oder 4C) umfasst, die nicht fluidisch miteinander verbunden sind, wobei:
- die Reaktionszone (4A) eine Zone zur Visualisierung der Assayergebnisse (5) umfasst, die mindestens einen zweiten immobilisierten Verbindungspartner umfasst, der imstande ist, sich mit dem mindestens einen Analyten zu verbinden, und
- die Reaktionszone (4B oder 4C) eine Kontrollzone (6) umfasst, die es ermöglicht, das gute Funktionieren der Vorrichtung zu kontrollieren und die mindestens ein Analogon des mindestens einen Analyten umfasst, das imstande ist, sich mit dem mindestens einen markierten Verbindungspartner zu verbinden;
- wobei die Zone zum Auftragen der flüssigen Probe (2), die Markierungszone (3) und die Reaktionszone (4) fluidisch verbunden sind.

2. Vorrichtung nach Anspruch 1, wobei der erste Verbindungspartner und der zweite Verbindungspartner ausgewählt sind aus der Gruppe bestehend aus Antikörpern, Antikörpergemisch, Antikörperfragment, Antikörperfragmentgemisch, Antikörperanalogon, Antikörperanalogagemisch, Antigen, Antigengemisch, Protein, Proteingemisch, Polypeptid, Polypeptidgemisch, Peptid, Peptidgemisch.

3. Vorrichtung nach Anspruch 1, wobei die Kontrollzone (6) ferner ein Reagens zum Einfangen des Analogons umfasst, das auf der Matrix immobilisiert ist, und mit dem sich das Analogon verbinden kann.

4. Vorrichtung nach Anspruch 1, wobei in der Zone (6) das Analogon direkt oder indirekt auf der Matrix immobilisiert ist.

5. Vorrichtung nach Anspruch 3, wobei das Reagens zum Einfangen des Analogons ein Reagens ist, das mit dem zweiten Verbindungspartner der Zone zur Visualisierung der Ergebnisse des Assays identisch ist.

6. Vorrichtung nach Anspruch 4, wobei das Analogon des Analyten auf der Matrix mit einem Reagens, ausgewählt aus der Gruppe bestehend aus Antikörpern, Antikörpergemisch(en), Antikörperfragment, Antikörperfragmentgemisch, Antikörperanalogon, Antikörperanalogagemisch, Antigen, Antigengemisch, Protein, Proteingemisch, Polypeptid und Polypeptidgemisch, Peptid, Peptidgemisch, Rezeptor, Biotin/Steptavidin, Biotin/Avidin, immobilisiert ist.

7. Vorrichtung nach Anspruch 1, wobei das Analogon des Analyten ausgewählt ist aus der Gruppe, bestehend aus Antikörpern, Antikörpergemisch, Antikörperfragment, Antikörperfragmentgemisch, Antikörperanalogon, Antikörperanalogagemisch, Antigen, Antigengemisch, Protein, Proteingemisch, Polypeptid, Polypeptidgemisch, Peptid, Peptidgemisch.

8. Vorrichtung nach Anspruch 1, wobei
(i) der erste Verbindungspartner ein Antikörper, ein Antikörperfragment oder ein Antikörperanalogon ist, der zweite Verbindungspartner ein Antikörper, ein Antikörperfragment oder ein Antikörperanalogon ist und das Analogon des Analyten ein Protein, ein Polypeptid oder ein Peptid ist, oder
(ii) der erste Verbindungspartner ein Protein, ein Polypeptid oder ein Peptid ist, der zweite Verbindungspartner ein Protein, ein Polypeptid oder ein Peptid ist, und das Analogon des Analyten ein Antikörpers, ein Antikörperfragment oder ein Antikörperanalogon ist.

9. Vorrichtung nach Anspruch 8, wobei das Analogon des Analyten auf der Matrix durch ein Einfangreagens immobilisiert ist, bei dem es sich um ein Reagens handelt, das mit dem zweiten Verbindungspartner identisch ist.

10. Vorrichtung nach Anspruch 1, wobei der erste Verbindungspartner mit einem nachweisbaren Marker markiert ist.

11. Vorrichtung nach Anspruch 10, wobei der nachweisbare Marker eine Verbindung, eine Substanz oder ein Partikel ist, die/der mit visuellen, fluoreszierenden, instrumentellen Mitteln nachgewiesen werden kann, und wobei der nachweisbare Marker insbesondere ein Partikel aus farbigem Latex, ein Goldpartikel, ein magnetischer Partikel sein kann.

12. Vorrichtung nach einem der vorhergehenden Ansprüche, wobei das gute Funktionieren der Vorrichtung und des Assays in Form einer nachweisbaren Linie sichtbar gemacht wird, die im Wesentlichen senkrecht zur Flussrichtung der flüssigen Probe verläuft.

13. Vorrichtung nach den vorhergehenden Ansprüchen, die außerdem eine Kontrolle der Migration der flüssigen Probe (7) umfasst.

14. Verfahren zum Ausführen eines Assays zur Bestimmung der Gegenwart oder der Abwesenheit eines Analyten in einer flüssigen Probe, das die folgenden Schritte umfasst:
Inkontaktbringen der flüssigen Probe mit einer Vorrichtung, wie in einem der vorhergehenden Ansprüche definiert, und Bestimmen, ob die Probe den Analyten umfasst oder nicht umfasst, durch Bezugnahme auf die Abwesenheit oder die Gegenwart eines nachweisbaren Signals an der Zone zur Visualisierung der Ergebnisse des Assays, und wenn an der Zone zur Visualisierung der Ergebnisse des Assays kein Signal vorhanden ist, Bestätigen des guten Funktionierens des Assays durch Bezugnahme auf die Gegenwart eines Signals an der Kontrollzone.

## Claims

1. Device for carrying out an assay to determine the presence or the absence of at least one analyte in a liquid sample, comprising:
- a) a support,
- b) a matrix (1), attached to the support, which allows the migration of the liquid sample, said matrix comprising:
- (i) a zone of application of the liquid sample (2),
- (ii) a labelling, zone (3) comprising at least one first, labelled binding partner which is capable of binding to said at least one analyte, if it is present in the liquid sample, and which is capable of binding to at least one analogue of the analyte, and
- (iii) at least one reaction zone (4) comprising two adjacent and parallel reaction zones (4A and 4B or 4C) which are not in fluidic communication with one another, in which:
- the reaction zone (4A) comprises a zone for visualizing the results of the assay (5), comprising at least one second, immobilized binding partner which is capable of binding to said at least one analyte, and
- the reaction zone (4B or 4C) comprises a control zone (6) which makes it possible to control the correct operation of the device and which comprises at least one analogue of said at least one analyte which is capable of binding to said at least first, labelled binding partner;
- said zone of application of the liquid sample (2), labelling zone (3) and reaction zone (4) being in fluidic communication.

2. Device according to Claim 1, in which the first binding partner and the second binding partner are chosen from the group consisting of an antibody, an antibody mixture, an antibody fragment, an antibody fragment mixture, an antibody analogue, an antibody analogue mixture, an antigen, an antigen mixture, a protein, a protein mixture, a polypeptide, a polypeptide mixture, a peptide and a peptide mixture.

3. Device according to Claim 1, in which the control zone (6) also comprises a reagent for capturing the analogue, immobilized on the matrix, and to which said one analogue is capable of binding.

4. Device according to Claim 1, in which, in the zone (6)', the analogue is immobilized on the matrix directly or indirectly.

5. Device according to Claim 3, in which the reagent for capturing the analogue is a reagent which is identical to the second binding partner of the zone for visualizing the results of the assay.

6. Device according to Claim 4, in which the analogue of the analyte is immobilized on the matrix by means of a reagent chosen from the group consisting of an antibody, an antibody mixture or antibody mixtures, an antibody fragment, an antibody fragment mixture, an antibody analogue, an antibody analogue mixture, an antigen, an antigen mixture, a protein, a protein mixture, a polypeptide and a polypeptide mixture, a peptide, a peptide mixture, a receptor, biotin/steptavidin, and biotin/avidin.

7. Device according to Claim 1, in which the analogue of the analyte is chosen from the group consisting of an antibody, an antibody mixture, an antibody fragment, an antibody fragment mixture, an antibody analogue, an antibody analogue mixture, an antigen, an antigen mixture, a protein, a protein mixture, a polypeptide, a polypeptide mixture, a peptide and a peptide mixture.

8. Device according to Claim 1, in which
(i) the first binding partner is an antibody, an antibody fragment or an antibody analogue, the second binding partner is an antibody, an antibody fragment or an antibody analogue, and the analogue of the analyte is a protein, a polypeptide or a peptide, or
(ii) the first binding partner is a protein, a polypeptide or a peptide, the second binding partner is a protein, a polypeptide or a peptide, and the analogue of the analyte is an antibody, an antibody fragment or an antibody analogue.

9. Device according to Claim 8, in which the analogue of the analyte is immobilized on the matrix by means of a capture reagent which is a reagent identical to the second binding partner.

10. Device according to Claim 1, in which the first binding partner is labelled with a detectable label.

11. Device according to Claim 10, in which the detectable label is a compound, a substance or a particle which can be detected by visual, fluorescent or instrumental means, and in particular the detectable marker may be a coloured latex particle, a gold particle or a magnetic particle.

12. Device according to any one of the preceding claims, in which the correct operation of the device and of the assay is embodied in the form of a detectable line which is substantially perpendicular to the direction of the flow of the liquid sample.

13. Device according to the preceding claims, also comprising a liquid-sample migration control (7).

14. Method for carrying out an assay to determine the presence or absence of an analyte in a liquid sample, comprising the steps of:
bringing the liquid sample into contact with a device as defined ism any one of the preceding claims and determining whether or not the sample comprises the analyte by reference to the absence or to the presence of a detectable signal at the level of the zone for visualizing the results of the assay and, when there is no signal at the level of the zone for visualizing the results of the assay, confirming the correct operation of the assay by reference to the presence of a signal at the level of the control zone.
